# EUROPÄISCHE PATENTSCHRIFT

(11) **EP 0 707 064 B1**
(45) Veröffentlichungstag und Bekanntmachung des Hinweises auf die Patenterteilung: **24.10.2001**
(21) Anmeldenummer: 95890148.0
(22) Anmeldetag: 09.08.1995
(51) Int. Cl.: C12N 11/04, C12N 11/18, C12Q 1/00

(54) **Verfahren zur Immobilisierung biologischer Komponenten in einer Polymermatrix sowie Biosensoren unter Verwendung derartiger Immobilisate**
Process for immobilizing biological components in a polymeric matrix as well as biosensors obtained therefrom
Procédé d'immobilisation d'agents biologiques dans une matrice polymerique ainsi que des biosenseurs utilisant de tels immobilisats

(30) Priorität: 05.10.1994 AT 189394
(43) Veröffentlichungstag der Anmeldung: 17.04.1996
(73) Patentinhaber: F. Hoffmann-La Roche AG, 4070 Basel (CH)
(72) Erfinder: Offenbacher, Helmut, Dr., A-8020 Graz (AT); Schaffar, Bernhard, Mag.Dr., A-8045 Graz (AT); Ghahramani, Massoud, Dr., A-8102 Semriach (AT)
(74) Vertreter: Babeluk, Michael, Dipl.-Ing. Mag.

(56) Entgegenhaltungen:
- EP-A- 0 195 304
- EP-A- 0 228 259
- EP-A- 0 297 912
- WO-A-89/07139
- WO-A-92/04465
- DE-A- 2 629 693
- GB-A- 1 469 358
- US-A- 4 250 267
- US-A- 4 269 941

## Beschreibung

Die Erfindung bezieht sich auf ein Verfahren zur Immobilisierung von biologischen Komponenten, vorzugsweise von Enzymen, in einer Polymermatrix, auf ein mit diesem Verfahren hergestelltes Immobilisat, sowie auf Biosensoren unter Verwendung dieses Immobilisates.

Für die Immobilisierung von Enzymen sowie anderen reaktiven biologischen Komponenten und die damit verbundene Nutzbarmachung in diagnostischen Sensorelementen gibt es eine Reihe von Methoden, die im folgenden kurz dargestellt werden.

Folgende Immobilisierungsmethoden sind bis dato bekannt und werden je nach Art der Problemstellung technologisch genutzt:
A) Immobilisierung der biologischen Komponenten durch chemische Bindung
   1) an ein Trägermaterial
   2) durch Quervernetzen mit bifunktionellen Vernetzern
   3) in Polymernetzwerken
B) Adsorptive Bindung der biologischen Komponenten an aktiven Oberflächen
C) Immobilisierung der biologischen Komponente durch physikalische Einbettungsverfahren
D) Hybridverfahren

Im folgenden wird nur auf die verschiedenen Methoden der Immobilisierung durch physikalische Einbettung gemäß Punkt C) in puncto Attraktivität sowie verfahrensimmanenter Problematik näher eingegangen, da die vorliegende Erfindung in diesem Bereich einzuordnen ist.

Bei dieser Art der Immobilisierung werden biologische Komponenten z. B. Enzyme, in makroskopischen bis molekularen Cavitäten einer Einbettungsmatrix eingebracht.

Folgende Methoden sind bekannt:
a) Einlagerung eines Enzymes zwischen zwei für das Enzym impermeablen Membranfolien (z.B.Cellophan).
b) Bereitung einer Emulsion von Enzymlösungen in einem vernetzbaren Polymer - die Enzymlösung liegt nach dem Vernetzen in der Polymermatrix in Tröpfchenform vor.
c) Enzymkristallsuspension in vernetzten bzw. nichtvernetzten Polymeren.
d) Enzymmikrodomänen in einem nichtvernetzten Polymer, herstellbar durch Lösen von Enzymen in Polymer-Wasser-Latices, wobei das zumeist hochmolekulare Polymer durch Zusatz von weichmacherähnlichen Verfilmungshilfen beim Trocknen zu einer weitgehend, von Enzym-Mikrotröpfchen durchsetzten, homogenen Thermoplastschichte umgewandelt wird (siehe beispielsweise Polymerdispersion in WO 89/07139).
e) Inkorporierung der biologischen Komponente durch Migration in eine gequollene Harzmatrix (Poly HEMA, Polyacrylamidvernetzt)
f) Inkorporierung der biologischen Komponente durch Polymerfällung in einem wäßrigen Enzym-Polymersystem
g) Immobilisierung der biologischen Komponente durch Einbringen von Enzymen in ein durch Polymerisation vernetzbares wasserlösliches Hydrophilpolymer (z. B. UV-härtender Polyvinylalkohol)
h) Einkapselung der biologischen Komponente in Nanokapseln sowie Zellghosts etc.

Diese Verfahren haben je nach Anwendung und Art der biologischen Komponente Vor- und Nachteile. Als generell nachteilig erwiesen sich für:
a) Abdichtproblematiken sowie homogene Enzymverteilung im Spalt, Fixierung am Transducer sowie Abhängigkeit von Folieneigenschaften
b,c) Inaktivierung der Enzyme durch Vernetzungshilfen wie Katalysatoren, Monomere u. a., ferner sind die für die Einbettung in Frage kommenden Polymermaterialien für hydrophile Substrate schlecht permeabel.
d) Für diese Art der Immobilisierung gelten die selben Nachteile wie in b) und c) angeführt, ferner kommt es bei Langzeitflüssigkontakt zu einer mehr oder weniger starken Reversion des Verfilmungsprozesses, d. h. die Polymerschichte vertrübt sich, wird für das Biopolymer permeabel und wandelt sich in weiterer Folge wieder in eine Latexsuspension um.
e,f) Die Quellung begünstigende Lösungsmittel führen in der Regel zu einer Enzyminaktivierung bzw. da derartige Polymere von Haus aus polar sein müssen, besitzen sie selbst im Wasser eine in Bezug auf Enzymmigration nicht unbedenkliche Quellung. Da es sich bei den hiefür verwendeten Polymeren um nichtvernetzte Systeme handelt, tritt Migration bereits durch Umorientierung der Molekülketten im Zuge einer Matrixrelaxation ein.
g) Die für die Vernetzungsreaktion benötigten Radikalbildner bzw. die für den Start der Vernetzung benutzte energiereiche Strahlung führt zu einer starken Beeinträchtigung der Enzymaktivität. Die in diesem Zusammenhang bisher bekannten hydrophilen Polymere - es handelt sich dabei vor allem um modifizierte Polyvinylalkohole - besitzen selbst im vernetzten Zustand eine extrem starke Quellung, ferner ist der Vernetzungsgrad vom Wassergehalt des Enzympräpolymerfilms abhängig.
h) Diese Verfahren sind zwar literaturbekannt, erfordern jedoch einen hohen technologischen Aufwand.

Langzeitstabile Biosensoren benötigen Enzymschichten, die zum einen eine hohe Aktivität besitzen, zum anderen eine gute Stabilität in Bezug auf Naßlagerung aufweisen. Demnach ergibt sich für die Immobilisierung biologischer Komponenten folgender Forderungskatalog:
1) Einbringbarkeit großer Mengen an biologischer Komponente in eine derartige Reaktivschichte
2) Kein (bzw. nur geringfügiger) Aktivitätsverlust bei der Immobilisierung, d. h. Vermeidung von reaktiven Vernetzern, Radikalbildnern, Schwermetallkatalysatoren sowie Vermeidung von beeinträchtigenden Monomeren und Lösungsmitteln.
3) Immobilisierung unter schonenden Bedingungen, d. h. bei möglichst niederer Temperatur sowie Vermeidung energiereicher Strahlung
4) Gewährleistung einer möglichst hohen Analytpermeabilität bei gleichzeitiger Minimierung der Enzymmigration
5) Aufrechterhaltung der für die Funktion des Enzyms optimalen Tertiärstruktur
6) Ausschließen einer mikrobiellen Befallbarkeit
7) Gewährleistung der Kompatibilität mit biologischen Medien
8) Minimierung von Haftproblematiken (Enzymimmobilisat - sensitive Einheit), hohe Reproduzierbarkeit der Immobilisierung
9) Gewährleistung einer einfachen Herstellungstechnologie
10) Modifizierungsfähigkeit dieser Herstellungstechnologie

Aufgabe dieser Erfindung ist es, möglichst allen angeführten Forderungen weitgehend gerecht zu werden.

Diese Aufgabe wird erfindungsgemäß dadurch gelöst,daß zumindest eine biologische Komponente mit einem wasserlöslichen oder in Wasser ohne Hilfsmittel emulgierbaren Präpolymer vermischt wird, welches eine zumindest über weite Sequenzen unpolare Hauptkette aufweist, an welcher direkt oder in Seitenketten polare, hydrophile Gruppen anhaften, und daß das mit der biologischen Komponente vermischte Präpolymer bei Temperaturen von Raumtemperatur bis 70°C, vorzugsweise bis 40°C über vernetzungsfähige Gruppen nach Abdunsten des wäßrigen Lösungsmittels zu einer dreidimensional vernetzenden, hydrophoben Polymermatrix reagiert, in welche die biologische Komponente eingebettet ist.

Insbesondere ist erfindungsgemäß vorgesehen, daß ein Präpolymer aus der Gruppe der Polykondensations-, Polyadditions- und Polymerisationsharze verwendet wird, dessen Hauptkette zur Gruppe der Polyester, Polyamide, Epoxyharze, Phenolharze, Polyacrylate und Polymethacrylate gehört, deren polare, hydrophile Gruppen der Gruppe der Carboxylat-, Amino-, Ammonium-, Hydroxyl- und Alkoxylgruppen angehören und deren vernetzungsfähige Gruppe aus ein- bis mehrfach ungesättigten Fettsäureestern, β-Diketo-Gruppen, sekundären Aminogruppen, geschützten Isocyanatgruppen, Epoxygruppen, Silanol- und Estergruppen bestehen, sowie daß allfällige polare Sequenzen der Hauptkette zur Gruppe der Polyoxyalkylene, vorzugsweise Polyoxyethylen der polymerisierten Acryl- und Methacrylsäuren sowie der Hydroxyalkylacrylate bzw. - methacrylate gehören.

Zum Unterschied von quervernetzenden Hydrophilpolymeren gemäß Punkt g) bestehen die in der Erfindung genannten Oligomere bzw. Präpolymere aus einer unpolaren bzw. über weite Sequenzen unpolaren Polymerhauptkette, an welcher sich direkt oder an kürzeren der Hauptkette anhaftenden tentakelartigen Seitenketten gerade soviele ionische (quarternäre Ammonium-, Amin- bzw. Carboxylatgruppen) bzw. polare (-OH, -O[-(CR₂)ₙ-O]ₘ - (n = 2 - 5,m = 1 - 100, R = H, Alkyl, Acryl) u. a. Gruppen befinden, daß das Präpolymer gerade noch wasserlöslich, wasserverdünnbar bzw. in Wasser ohne Hilfsmitteln emulgierbar ist, sowie Gruppen enthält, die unter milden Bedingungen bereits ohne Katalysatoren und ohne Anwendung von energiereicher Strahlung nach Abdunsten des Lösungsmittels von sich aus zu einem schwach polaren hydrophoben in Wasser schlecht bis mäßig quellenden dreidimensional vernetzten Matrixpolymer reagieren.

An der Vernetzung selbst sollte die biologische Komponente nicht beteiligt sein, je nach Vernetzungstyp ist es erfindungsgemäß jedoch möglich, daß in die Vernetzungsreaktion der Präpolymere auch periphäre, die Funktion der biologischen Komponente nicht beeinträchtigende Bereiche der biologischen Komponente einbezogen werden. Es können auch Salzbrücken zwischen dem Biopolymer und der ionische Gruppen tragenden Polymermatrix ausgebildet werden und einen biopolymerstabilisierenden Effekt bewirken.

Ein Immobilisat, bestehend aus in einer Polymermatrix immobilisierten biologischen Komponenten, vorzugsweise Enzymen, ist somit erfindungsgemäß dadurch gegeben, daß das der Polymermatrix zugrundeliegende Präpolymer wasserlöslich oder in Wasser ohne Hilfsmittel emulgierbar ist und eine zumindest über weite Sequenzen unpolare Hauptkette aufweist, an welcher direkt oder in Seitenketten polare hydrophile Gruppen anhaften, sowie daß das Präpolymer über vernetzungsfähige Gruppen verfügt, welche nach der Polymerisation ein dreidimensionales Netzwerk bilden, in welches die biologische Komponente eingebettet ist.

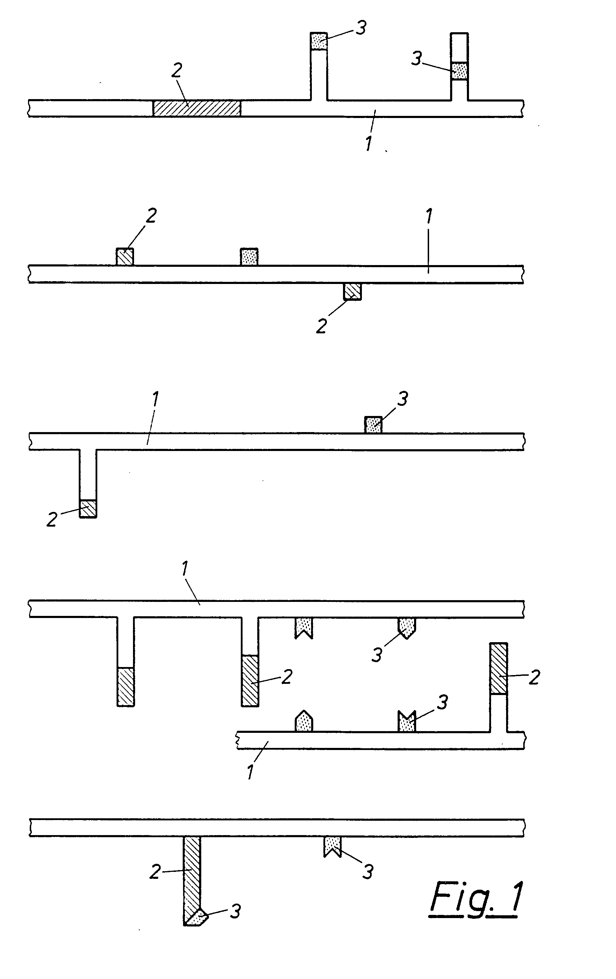

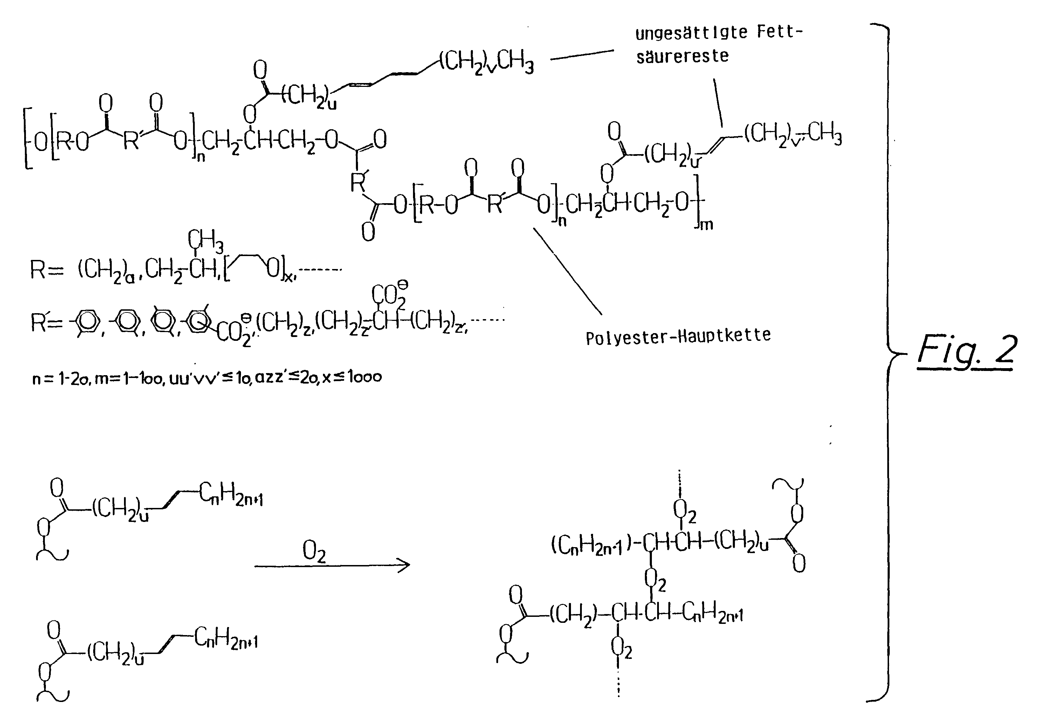

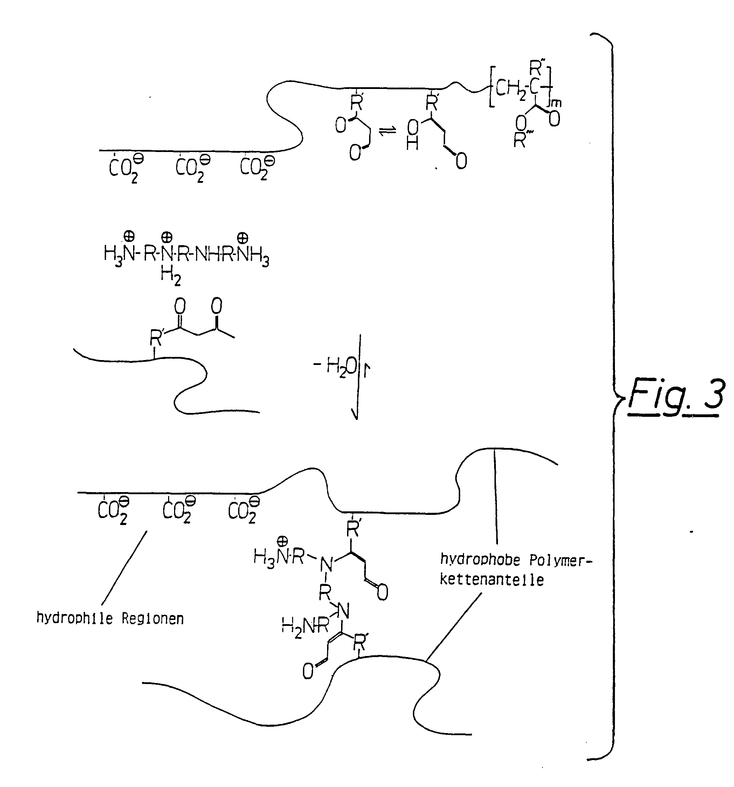

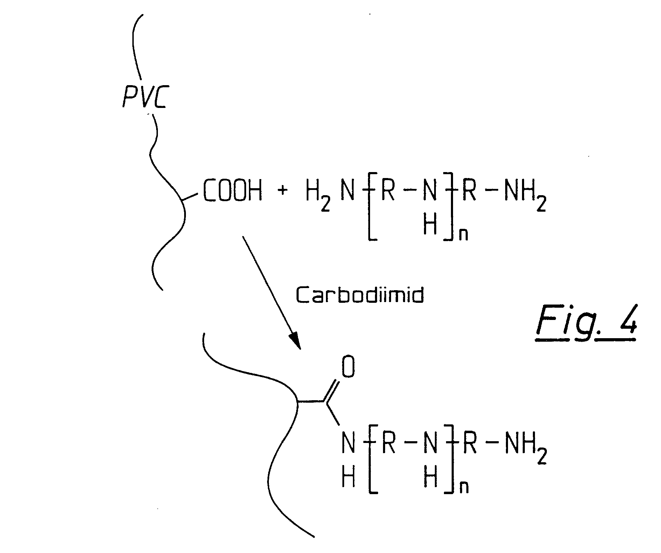

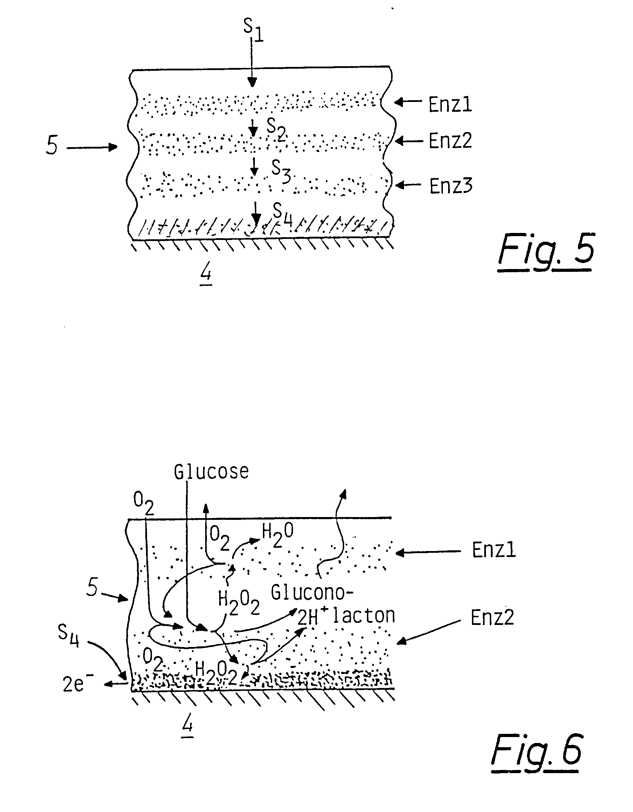

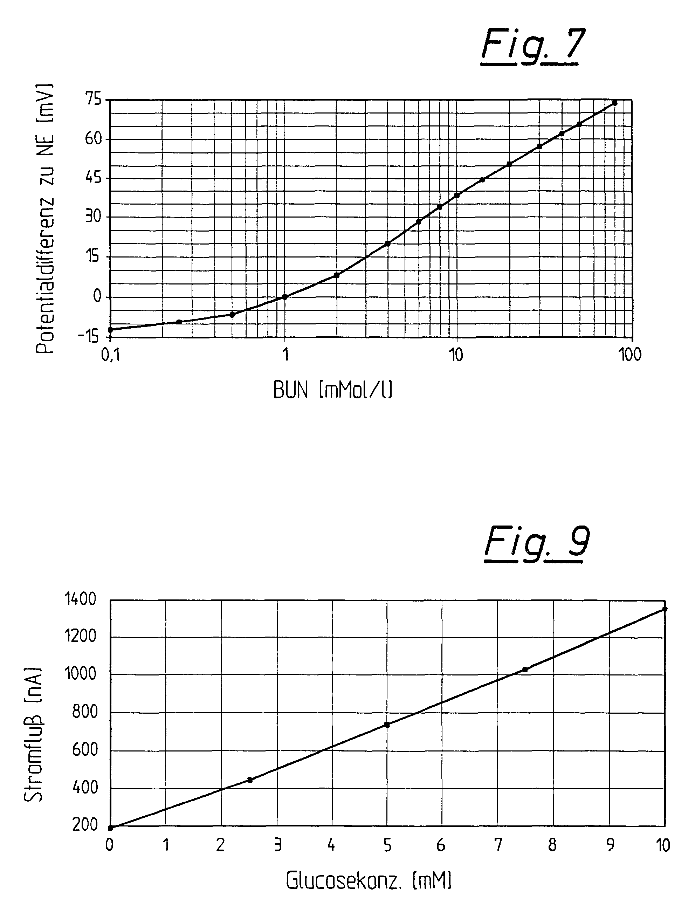

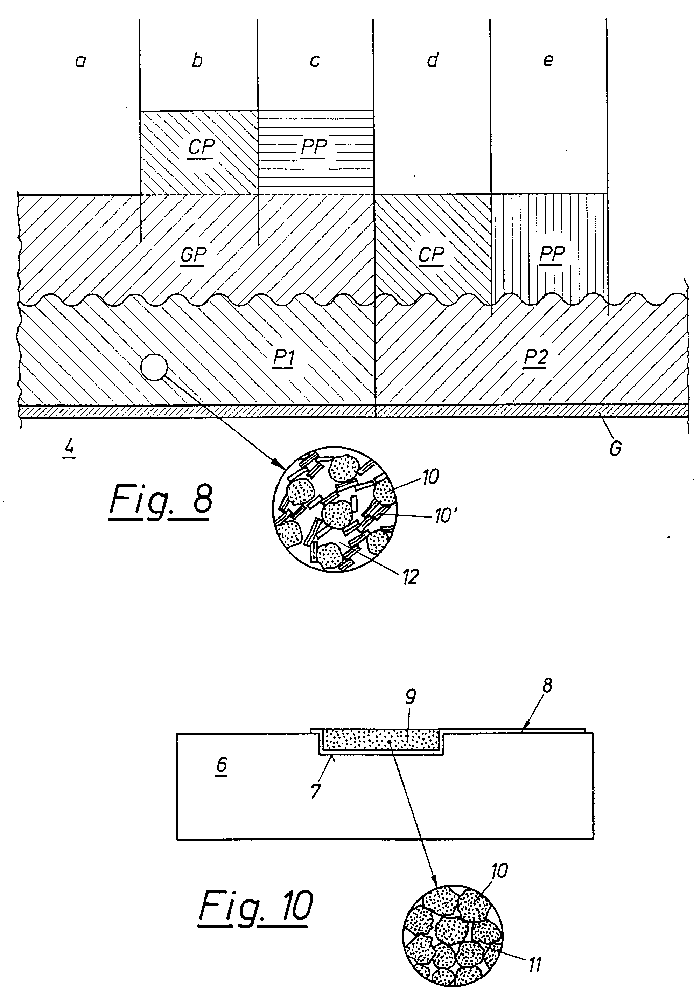

Zum besseren Verständnis der Erfindung wird auf folgende Abbildungen verwiesen. Es zeigen Fig. 1 für das erfindungsgemäße Verfahren geeignete Präpolymere in schematischer Darstellung, Fig. 2 die Vernetzungsreaktion von geeigneten Präpolymeren via Luftsauerstoff, Fig. 3 die Vernetzungsreaktion von enaminvernetzenden Präpolymeren, Fig. 4 eine Oberflächenmodifizierung von PVC, Fig. 5 einen erfindungsgemäßen, schichtartig aufgebauten Biosensor, Fig. 6 eine Ausführungsvariante nach Fig. 5 (Glukosesensor), Fig. 7 die Kennlinie eines erfindungsgemäß hergestellten BUN (blood urea nitrogen) Sensors, Fig. 8 Sensorbeispiele a bis e, Fig. 9 die Kennlinie eines erfindungsgemäß hergestellten Glucosesensors, wobei der Strom (nA) über der Glucosekonzentration aufgetragen ist, und Fig. 10 eine weitere Ausführungsvariante eines erfindungsgemäßen Biosensors.

Es hat sich gezeigt, daß sich für diese Art der Biopolymerimmobilisierung z.B. wasserlösliche vernetzbare Lackkunstharze, deren molekularer Aufbau in Fig. 1 schematisch dargestellt ist, besonders gut eignen. In dieser Darstellung sind die hydrophoben Oligomerkettenanteile mit 1, die hydrophilen Sequenzen mit 2 und die bezüglich der Vernetzung reaktiven Gruppen mit 3 bezeichnet. Diese Substanzklasse weist Eigenschaften auf, die den erfindungsgemäßen Präpolymereigenschaften entsprechen, d. h. sie sind wasserlöslich bzw. wasserverdünnbar, verfilmen nach Abdunsten des Wassers und vernetzen in weiterer Folge nach Verlust ihres Lösungsmittels ohne fremdes Zutun bzw. unter milden Bedingungen in Gegenwart von Luftsauerstoff bzw. bei schwach erhöhter Temperatur zu einem mechanisch stabilen, wasserbeständigen nur schwach bis mäßig quellenden, an sich hydrophoben Polymer.

Beim Trocknungs- und Vernetzungsprozeß behält das Polymer gerade soviel Wasser, daß die biologische Komponente in optimal hydratisierter Form, d. h. in Form seiner optimalen Wirksamkeit inkorporiert wird. Etwaige periphäre Fixierungen schmälern die Aktivität dieser Komponente nicht, vielmehr tragen sie dazu bei, daß die Migrationsneigung selbst bei weitmaschigen Vernetzungsstrukturen zusätzlich reduziert wird. Dabei ist es erfindungsgemäß von Vorteil, wenn der Moleküldurchmesser der biologischen Komponente um mindestens einen Faktor 3, vorzugsweise um einen Faktor > 10 größer ist als die Maschenweite der dreidimensional vernetzten Polymermatrix, sodaß die biologische Komponente migrationshemmend in der Polymermatrix zurückgehalten wird.

Zu den erfindungsgemäßen Oligomer- bzw. Präpolymersystemen sind folgende Substanzklassen zu zählen:

Fettsäuremodifizierte Polyester (Ölalkydharze), deren Hauptkette eine Poyesterkette darstellt, an der via Esterbildung ein- bis mehrfach ungesättigte Fettsäuren tentakelartig geknüpft sind. Die Wasserlöslichkeit bzw. Wasseremulgierbarkeit wird durch Einbau ionischer bzw. polarer Gruppen in die Hauptkette bzw. durch Insertieren hydrophiler Sequenzen in die Hauptkette erzielt. Die Vernetzung geschieht via Luftsauerstoff, wobei die Fettsäureseitenketten intermolekular über Peroxidbrücken verknüpft sind. Für die Schaffung der einzelnen Strukturelemente können folgende Ausgangssubstanzen verwendet werden: Bifunktionelle Säuren wie Phthalsäureanhydrid, Terephthalsäure, Isophthalsäure, bifunktionelle aliphatische Säuren wie Bernstein-, Adipin- sowie Sebazinsäure, u. a. bifunktionelle Alkohole wie Ethylenglycol, Phopylenglykol, Neopentylglykol, Biophenol A, Butylenglykol, Di- und Triethylenglykol, Polyethylenglykol, Polypropylenglykol, Monoglyceride ein bis mehrfach ungesättigte Fettsäuren, Umesterungsprodukte von Lein-, Rizinen-, Holz-, Tallölen und anderen natürlichen sowie synthetischen Fettsäuretriglyceriden mit mehrfunktionellen Alkoholen wie Pentaerithrit, organische Säuren mit einer Funktionalität ≥ 3 sowie Fruchtsäuren u. a.

Das Bauprinzip sowie die Vernetzungsreaktion ist in Fig. 2 dargestellt.

Bei der beschriebenen Vernetzung mit Sauerstoff kann erfindungsgemäß bei Erhöhung des Sauerstoffpartialdruckes auf Werte über 250 mbar, vorzugsweise auf Werte über 500 mbar, die Vernetzungstemperatur unter Raumtemperatur, vorzugsweise auf Temperaturen von 0 bis 5°C abgesenkt werden. Dies kann bei der Einbettung temperaturempfindlicher Enzyme von großem Vorteil sein.

In einer weiteren Ausführungsvariante der Erfindung ist vorgesehen, daß als Präpolymer enaminvernetzende Präpolymere, vorzugsweise Polyacrylate oder Polymethacrylate verwendet werden, welche durch Abdunsten des Lösungsmittels Wasser vernetzt werden.

Enaminvernetzende Polymere sind im wesentlichen mit sekundären mehrfunktionellen Aminen neutralisierte carboxylat- und β-diketogruppentragende Polyacrylate bzw. Polymethacrylate, bei denen das Oligomer in Form einer wäßrigen Emulsion vorliegt. Diese daraus gefertigten Oligomer-Biopolymerschichten verfilmen beim Abdunsten des Lösungsmittels zu einer porenfreien dichten Matrix, welche bei Raum- bzw. leicht erhöhter Temperatur (50°C) zu einem transparenten schwach quellenden (die Gewichtszunahme nach 2 Stunden Naßlagerung beträgt maximal 15%) Polymerfilm mit eingelagerter biologischer Komponente vernetzt. Das Vernetzungsschema ist in Fig. 3 dargestellt.

Als biologische Komponente kann bevorzugt ein bzw. mehrere Enzyme aus der Gruppe der Oxidoreduktasen, vorzugsweise Glucoseoxidase (EC 1.1.3.4), Lactatoxidase (EC 1.1.3.2) oder Ascorbatoxidase (EC 1.10.3.3); ein Enzym aus der Gruppe der Hydrolasen, vorzugsweise Urease (EC 3.5.1.5), Kreatinase (EC 3.5.3.3) oder Kreatininase (EC 3.5.4.21) eingesetzt werden.

Es ist leicht ersichtlich, daß sowohl Vernetzungsgrad als auch Quellfähigkeit in Richtung guter Substratpermeabilität bei minimaler Migration der biologischen Komponente über den Gehalt an vernetzungsfähigen Regionen bzw. Gruppen sowie über das Verhältnis polare zu unpolare Oligomerkettensequenzen steuerbar, also über die Maschenweite und Hydratisierbarkeit sowohl der Polymerkette als auch der Vernetzungsbrücken beeinflußbar ist.

Zur großen Familie der wasserverdünnbaren bzw. mit Wasser dispergierbaren Oligomere, die im Zuge einer milden Vernetzungsreaktion hydrophobisiert werden, gehören ferner:

Silikonharzmodifizierte Ölalkydharze = Fettsäuremodifizierte Polyester-Silikon-Copolymerisate, Isocyanatharze mit geschützter Isocyanatgruppe, Epoxyharze mit hydrolysegeschützter Epoxyfunktionalität sowie sämtliche Hybridharze, deren Merkmale jenen der für die erfindungsgemäße Immobilisierung von Biopolymeren befähigten Oligomeren entsprechen.

Wie bereits erwähnt, finden die Vernetzungsreaktionen nach Verlust des Lösungsmittels, d. h. im Zuge der Annäherung der reaktiven Gruppen bereits bei Raumtemperatur statt, d. h., sie können, müssen jedoch nicht zusätzlich katalysiert werden. Im Falle der oxidativen Trocknung von Ölalkydharzen kann die Vernetzungsreaktion durch Erhöhung des Sauerstoffpartialdruckes sowie durch leichte Erhöhung der Reaktionstemperatur auf 40° bis maximal 60° C auch auf einfache Weise beschleunigt werden.

Kondensationsvernetzende Systeme, wie z. B. die enaminvernetzenden β-diketomodifizierten Poly(meth-)acrylate zeigen bei schwach erhöhter Temperatur eine deutlich forcierte Trocknung. Vakuumbehandlung bzw. Absenken der relativen Luftfeuchtigkeit wirken sich sowohl auf Geschwindigkeit als auch auf den Vernetzungsgrad positiv aus.

Ein generelles Problem bei der Entwicklung von Biosensoren mit Langzeitstabilität in wäßrigen Systemen ist die Haftung zwischen Immobilisat und Untergrund, wobei es sich beim Untergrund lediglich um eine Trägeroberfläche aber auch um die Oberfläche eines sensitiven Elementes handeln kann. Ein erfindungsgemäßer Biosensor zeichnet sich nun dadurch aus, daß der Sensor eine Trägerschicht aufweist, welche dahingehend chemisch modifiziert ist, daß diese zumindest eine dem Präpolymer des Immobilisates analog vernetzungsfähige Gruppe zum Zwecke der Haftverbesserung zwischen Immobilisat und Trägerschicht aufweist.

Im Falle von oxidativ trocknenden Präpolymeren kann eine Haftverbesserung z. B. dann erzielt werden, wenn man den Untergrund durch Immobilisierung von ungesättigten Fettsäuren (doppelbindungstragende Fettsäuren) via Ester oder Amid modifiziert.

Die Kombination von Enzymimmobilisatschichten mit ionensensitiven Elektroden ist dann sinnvoll, wenn bei der enzymatischen Umsetzung eines zum Beispiel klinisch relevanten Substrates ein Ion entsteht, welches mit einer geeigneten ionensensitiven Elektrode gemessen werden kann. In üblicher Weise besteht das sensitive Element derartiger Elektroden aus einer Mischung aus PVC, Weichmacher und geeigneten Ionophor. Will man eine derartige ionensensitive Membran in Bezug auf die Vernetzung mit dem Präpolymer der Enzymschichte funktionalisieren, so modifiziert man den PVC-Anteil der Membran, d. h. man verwendet statt normales PVC ein funktionalisiertes (z. B. carboxyliertes Polyvinylchlorid der Fa. Aldrich-Artikel No. 18,955-3) und modifiziert es gemäß den Anforderungen.

Es hat sich gezeigt, daß enaminvernetzende Oligomersysteme dann einen guten Haftverbund zu derartigen ionensensitiven Membranen eingehen, wenn man Carboxy-PVC mit Polyaminen der Klasse H₂N-(R-NH-)ₙ R-NH₂ wobei R = (CH₂)ₘ; n = größer 1 vornehmlich 1-3,m = 1 - 10) bzw. anderen mindestens eine sekundäre Aminogruppe enthaltenden Substanzklassen mit Carbodiimid umsetzt. Bei niederem Carboxylierungsgrad des PVC's kommt es durch derartige Modifizierungen zu keiner Beeinträchtigung der Elektrodeneigenschaften wie Selektivität, Membranwiderstand, Ionophorenauswaschbarkeit etc. (s. Fig. 4).

In einer Weiterbildung der Erfindung ist vorgesehen, daß mehrere schichtartig angeordnete Immobilisate vorgesehen sind, welche das gleiche Matrixmaterial jedoch unterschiedliche biologische Komponenten aufweisen. Versieht man ein weitgehend vernetztes Immobilisat mit einer weiteren, eine biologische Komponente erhaltenden Präpolymerschicht, so wächst dieses Präpolymer nach Abdunsten des Wassers im Zuge der einsetzenden Vernetzungsreaktion mit der unteren Schichte zu einer durchgehenden 3-dimensional vernetzten Polymermatrix zusammen.

Auf diese Weise kann man einen in Bezug auf das Einbettungspolymer homogenen Schichtverbund erzeugen, wobei das bzw. jedes der schichtartig angeordneten Immobilisate mehrere biologische Komponenten nebeneinander beinhalten kann. Durch diese Maßnahme gelingt es in einer Schichte mehrere biokatalytische bzw. reaktive Domänen räumlich getrennt anzuordnen. In zumindest einem dieser Immobilisate kann man auf diese Weise auch katalytisch aktive und/oder ladungsableitende Partikel wie Edelmetallkalloide, reaktive bzw. katalytisch wirkende anorganische Partikeln, Metallpigmente sowie lichtisolierende Pigmente aber auch Kohlenstoffpigmente wie Graphit, Aktivkohle, Glassy carbon, edelmetalldotierten Kohlenstoff etc. einbringen. Ziel einer derartigen Maßnahme ist die Schaffung von Schichten, die biokatalytische sowie für die Bestimmung eines Analyten relevanten bzw. elektrochemisch aktive aber auch elektronenleitende Domänen schichtartig übereinander positioniert enthalten.

Wie in Fig. 5 dargestellt, kann ein derartiger Biosensor auf einer Trägerschicht 4 mehrere Immobilisate S₁ bis S₄ aufweisen, welche nach deren Vernetzung eine einheitliche Polymermatrix 5 bilden. Die einzelnen Immobilisate weisen Enzyme Enz₁ bis Enz₃ auf, in das Immobilisat S₄ können katalytische und/oder ladungsableitende Partikel eingelagert sein.

Durch diese Maßnahme gelingt es Schichten zu erzeugen, in denen enzymkatalysierte Reaktionskaskaden bis hin zum eigentlichen Sensorelement ablaufen können, im Falle der amperometrischen Bestimmung von Substraten, wie z. B. Glucose (s. Fig. 6) ist es möglich, ein elektrokatalytisches Elektrodenelement, welches auf das bei der enzymatischen Reaktion entstehende Folgeprodukt sensitiv ist, in die Schicht S₄ mitzuintegrieren. (Enz₁ = Catalase und Enz₂ = GOD). Das Immobilisat S₄ beinhaltet Pt und Kohlepartikel.

Als besonders vorteilhaft hat sich ein Immobilisat mit einer biologischen Komponente erwiesen, welche zusätzlich ein elektrokatalytisches Elektrodenelement enthält. Zu diesem Zwecke wird ein wäßriges Oligomer samt Enzymsystem mit Platin und Kohlepartikeln so gefüllt, daß nach Abdunsten des Wassers und nach Vernetzung des Oligomeren eine leitfähige enzymtragende Schichte entsteht. In dieser Ausgestaltungsform ist das Enzym an der Kohle-Elektrodenoberfläche nicht adsorbiert gebunden, sondern in den Zwischenräumen des porösen Elektrodenmaterials im 3-dimensional vernetzten Trägerpolymer molekulardispers eingesiegelt. Eine besondere Ausführungsform hiefür ist die Tränkung sogenannter elektrokatalytischen Kohlepapiere, bei denen edelmetallhaltige Kohle (z. B. Glassy carbon) schichtartig auf einem Trägervlies aufgebracht ist (s. Beisp. 7).

Im folgenden sind einige Beispiele einer erfindungsgemäßen Enzymimmobilisierung dargestellt:

### Beispiel 1

### Sensorelement zur Bestimmung von Harnstoff in biologischen Medien, wie z. B. in Blut

Bei der enzymatischen Umsetzung von Harnstoff entsteht Ammoniak, demnach kann man einen BUN-sensitiven Sensor dahingehend herstellen, indem man auf einen pH- bzw. NH₄⁺ Sensor eine erfindungsgemäßes Urease-Immobilisat aufbringt. In diesem Beispiel soll die Kombination Ureaseimmobilisat und ammoniumsensitive Elektrode dargestellt werden.

### a) Herstellung eines mit sekundären Aminogruppen modifizierten PVC

10 g Carboxy-PVC (Carboxy-Gehalt 1,8% - Aldrich Chem.Corp.) werden in 150 ml THF (Flukapuriss. pa) gelöst und mit 600 mg Dicyclohexylcarbodiimid (Fluka) sowie 500 mg Bishexamethylentriamin (Merck) versetzt. Nach 48-stündigem Rühren bei Raumtemperatur wird die Reaktionslösung in 1000 ml Aceton eingebracht und das modifizierte Polymer durch Zusatz von 250 ml destilliertem Wasser gefüllt. Die Füllung wird abfiltriert, mit Aceton, Ethanol und dest. Wasser gewaschen und nach 2-stündigem Suspendieren in Ethanol abgenutscht. Nach Vakuumtrocknung erhält man ein beigegefärbtes flockiges Pulver.

### b) Herstellen der ammoniumsensitiven Membran

300 mg Amino-PVC werden mit 10 mg Nonactin, in 3,5 g Tetrahydrofuran (Fluka puriss. pa) gelöst und mit 660 mg Bis (1-butylpentyl)adipat versetzt.

Diese Membranflüssigkeit kann z.B. in das Membranfenster eines Elektrodengehäuses (siehe DE 2 854 444 C2) eingebracht bzw. mit einem planen leitenden Untergrund fest kontaktiert werden.

### c) Immobilisierung des Enzyms

In 1 ml physiologischen Phosphatpuffer (pH 7,4) werden 0,5 ml einer wäßrigen ketiminvernetzenden Polyacrylatemulsion (Harzgehalt = 40%) eingebracht und mit 100 mg Urease (von der Schwertbohne - 124 U/mg; Fluka) versetzt. Diese Mischung ist lagerstabil und kann einige Wochen bei 0 bis +4°C gelagert werden. Die Mischung wird mit einem Dispensor auf die ammoniumsensitive Membran aufgetropft und eine Woche bei Raumtemperatur oder 2 Tage bei 35 bis 40°C gelagert.

Der Enzymaktivitätstest zeigt, daß während des Vernetzungsprozesses ein Aktivitätsverlust von lediglich 10 bis 15 % zu beobachten ist, bei weiterer Naßlagerung in einem physiologischen Puffersystem konnte kein weiterer Aktivitätsverlust mehr festgestellt werden. Verglichen mit ammoniumsensitiven Membranen, die nichtmodifiziertes PVC als Membranpolymer beinhalten, zeigt der erfindungsgemäße Verbund aus Elektrodenmembran und Immobilisat selbst bei Naßlagerung im Puffermedium eine sehr gute Haftung, so kann die Immobilisatschichte von der ammoniumsensitiven Membran nur unter gleichzeitiger Zerstörung derselben entfernt werden. Die Wasseraufnahme durch Quellung der Immobilisatschichte ist mäßig und beträgt nach mehrstündiger Lagerung im physiologischen Puffersystem 14%. Dieses Faktum gewährleistet eine gute Ammoniumpermeabilität, aufgrund der Sensorstabilität kann auf eine echte Enzymimmobilisierung geschlossen werden. Fig. 7 zeigt eine Kennlinie einer erfindungsgemäßen BUN (blood urea nitrogen) Elektrode, wobei die BUN-Konzentration über der Potentialdifferenz aufgetragen ist.

### Beispiele 2 bis 6

### Sensorelement zur Bestimmung von Glukose in biologischen Medien

Bei der Umsetzung von Glukose in Gegenwart von Glukoseoxidase und bei Abwesenheit von Mediatoren entsteht Gluconolacton sowie Wasserstoffperoxid amperometrisch, welches wiederum anodisch zu Sauerstoff oxidiert werden kann. Der dabei fließende Strom ist ein Maß für den Glukosegehalt der Probe. Im folgenden seien einige Beispiele für die Anwendung eines aus mehreren Immobilisaten gemäß vorliegender Erfindung aufgebauten Sensorelementes dargestellt.

### Herstellung der Ausgangskomponenten:

### a) Herstellung von Leinölfettsäureamidohexylamin:

0,2 mol Leinölfettsäure (techn.) werden mit 0,2 mol 1,6-Diaminohexan (pa Merck) und 0,2 mol Dicydohexylcarbodiimid (Fluka) in einem Liter Ethylacetat unter Inertgas bei Raumtemperatur umgesetzt. Nach Beendigung der Reaktion wird der entstandene Dicyclohexylharnstoff abfiltriert und die organische Phase mit einer verdünnten Kaliumchloridlösung mehrmals gewaschen. Nach Trocknen über Na₂SO₄ wird unter Schutzgasbeaufschlagung solange eingeengt, bis ein bräunliches Öl zurückbleibt. Das Reaktionsprodukt wird über Calciumoxid unter Argon gelagert.

### b) Trägermaterial:

Mit einer Gold-Ableitbahn besputterte Polymethylmethacrylatplättchen werden mit 35 volumsprozentiger Schwefelsäure oberflächlich hydrolysiert und in einer das Trägerplättchen nicht zu stark anquellenden Lösungsmittelmischung mit Leinölfettsäureamidohexylamin und Dicydohexylcarbodiimid (Fluka) 24 Stunden bei Raumtemperatur und unter Inertgas umgesetzt. Die Plättchen werden mit Benzin gewaschen und sofort weiter verarbeitet.
c) 1g mit ungesättigten Fettsäuren modifizierter Polyester (Ölalkydharz der Fa. Vianova 40%ig in Wasser) werden mit 0,5 g Platin 10% auf Aktivkohle (Fluka) sowie mit 0,5 g Graphit (Merck) vermischt und mit soviel Wasser versetzt, daß eine mit einer Laborschwingmühle bearbeitbare Paste entsteht. Die aufgeschlossene Paste läßt sich bei Raumtemperatur unter Inertgas lagern.
d) 1g der Paste (C) wird mit 0,05 g Glukoseoxidase (EC 1.1.3.4-15.000-25.000 Units/g) vermischt und bei Raumtemperatur unter Inertgas gelagert.
e) 1g Ölalkydharz 40% in Wasser werden mit 0,05 bis 0,1 g GOD (EC 1.1.3.4-15.000-25.000 Units/g) vermischt und die entstandene Harz-Enzym-Lösung bei Raumtemperatur unter Inertgas gelagert.
f) 1g Ölalkydharz 40% in Wasser werden mit 0,05 g Catalase (EC 1.11.1.6-5.000 Units/g) vermischt und die so entstandene Harz-Enzymlösung im Kühlschrank unter Inertgas gelagert.
g) 0,5 g Kaliumhexachloroplatinat (IV) wird in etwa 10 ml Wasser gelöst und mit einem geeigneten Reduktionsmittel zu einer braunen Platinkolloidsuspension reduziert. Nach gründlichem Dialysieren wird die Kolloidsuspension im Verhältnis 1 : 2 mit dem Ölalkydharz vermischt und bei Raumtemperatur unter Schutzgas gelagert.

### Sensorbeispiele:

### Beispiel 2

Auf den Sensorträger 4 mit einer Goldableitung G, hergestellt gemäß Punkt b), wird mit einem 200-400*µ*m Spacer die Pastenformulierung P1 (gemäß c) so aufgebracht, daß die Schichte die Gold-Ableitbahn G kontaktiert. Nach einem mehrstündigen Trocknungsschritt bei Raumtemperatur wird die GOD-Präpolymermischung GP (nach Punkt e) in einer Schichtstärke von etwa 30 - 100*µ*m (Naßfilm) aufgebracht (Fig. 8a). Die Ausschnittsvergrößerung der Schicht P1 zeigt Pt-Kohle-Partikel 10, Graphitpartikel 10' und das vernetzte Polymer 12.

### Beispiel 3

Vorgangsweise wie bei Beispiel 2, nach Antrocknen der GOD-Präpolymerschichte GP werden etwa 30 - 100*µ*m (bezogen auf Naßfilm) der Catalase-Präpolymermischung CP (nach Punkt f) aufgebracht (Fig. 8b).

### Beispiel 4

Wie Beispiel 3, anstelle der Catalase-Präpolymerlösung wird die Platinkolloid-Präpolymermischung PP (nach Punkt g) aufgebracht (Fig. 8c).

### Beispiel 5 und 6

Die Paste P2 (gemäß Punkt d) wird auf das Trägerplättchen 4 analog Paste P1 in den Beispielen 2-4 aufgebracht und nach Antrocknen analog Beispiel 3 bzw. Beispiel 4 mit den Formulierungen CP bzw. PP überschichtet (Fig. 8d und 8e).

Sämtliche Sensorvarianten werden mehrere Stunden bis mehrere Tage in einer O₂-angereicherten Atmosphäre bzw. an Luft der oxidativen Trocknung unterworfen und nach vollständigem Vernetzen in einem physiologischen Puffer gelagert. Das zusätzliche Aufbringen der Catalase- bzw. Platinmetall-Kolloidschichte ermöglicht zum einen eine Prolongierung des linearen Bereiches, zum anderen wird - was Forderung für die in vivo Messung ist - verhindert, daß H₂O₂ in den Probenraum abdiffundiert.

In Fig. 9 ist ein Diagram Strom versus Glukosekonzentration in einem physiologischen Puffer dargestellt.

### Beispiel 7

Gemäß Fig. 10 wird ein Kunststoffträger 6 mit einer topfartigen Vertiefung 7 so mit Gold besputtert, daß die Oberfläche des vertieften Bereiches mit der Kontaktierungsstelle 8 des Sensorträgers eine elektrisch leitende Einheit wird. In diese Vertiefung 7 wird ein Scheibchen 9 bestehend aus Pt-Kohle auf einem Trägervlies eingepreßt. 100 mg Lactatoxidase v. Pediococusspecies (L 0638 Sigma - 30 Units/mg) werden in 1 ml physiologischen Phosphatpuffer gelöst und mit 0,5 ml enaminvernetzendem Polyacrylat-Präpolymer (40% in Wasser) vermischt.

Die eingepreßte Pt-Kohleelektrode wird mit der Enzym/Präpolymerlösung getränkt, bei Raumtemperatur getrocknet und vernetzt. In der Ausschnittsvergrößerung sind mit 10 die mikroporösen Pt-Kohle-Partikel und mit 11 das Enzympolymer bezeichnet.

Das resultierende Sensorelement (Fig. 10) zeigt in Bezug auf Stromfluß Linearität bis zu einer Lactatkonzentration von 15 mmol/l und besitzt, verglichen mit einem Sensorelement, bei dem die Lactatoxidase auf dem identen Pt-Kohlenstoff-Elektrodenmaterial adsorptiv gebunden ist, eine um den Faktor >5 höhere Betriebslebensdauer.

## Patentansprüche

1. Verfahren zur Immobilisierung von biologischen Komponenten, vorzugsweise von Enzymen, in einer Polymermatrix,
**dadurch gekennzeichnet**,
a) dass zumindest eine biologische Komponente in einem wässrigen Lösungsmittel mit einem wasserlöslichen oder in Wasser ohne Hilfsmittel emulgierbaren Präpolymer vermischt wird, wobei das Präpolymer folgende Komponenten aufweist:
• eine zumindest über weite Sequenzen unpolare Hauptkette aus der Gruppe der Polyester, Polyamide, Epoxyharze, Phenolharze, Polyacrylate und Polymethacrylate,
• polare, hydrophile Gruppen, welche direkt oder in Seitenketten an der Hauptkette anhaften, aus der Gruppe der Carboxylat-, Amino-, Ammonium-, Hydroxyl- und Alkoxylgruppen,
• vernetzungsfähige Gruppen aus ß-Diketo Gruppen, sekundären Aminogruppen, geschützten Isocyanatgruppen, Epoxygruppen, Silanol- und Estergruppen,
b) dass die Mischung gemäß Punkt a) Temperaturen von Raumtemperatur bis 70°C, vorzugsweise bis 40°C ausgesetzt wird, sodass das Präpolymer über die vernetzungsfähigen Gruppen ohne Anwendung von Katalysatoren oder energiereicher Strahlung vernetzt, und
c) dass nach Abdunsten des wässrigen Lösungsmittels eine dreidimensional vernetzte, hydrophobe Polymermatrix gebildet wird, in welche die biologische Komponente eingebettet ist.

2. Verfahren nach Anspruch 1, **dadurch gekennzeichnet, dass** in die Vernetzungsreaktion des Präpolymers auch periphere, die Funktion der biologischen Komponente nicht beeinträchtigende Bereiche der biologischen Komponente einbezogen werden.

3. Verfahren nach Anspruch 1 oder 2, **dadurch gekennzeichnet, dass** die Hauptkette polare Sequenzen der Gruppe der Polyoxyalkylene, vorzugsweise Polyoxyethylen, der polymerisierten Acryl- und Methacrylsäuren sowie der Hydroxyalkylacrylate bzw. - methacrylate aufweist.

4. Verfahren zur Immobilisierung von biologischen Komponenten, vorzugsweise von Enzymen, in einer Polymermatrix,
**dadurch gekennzeichnet,**
a) dass zumindest eine biologische Komponente in einem wässrigen Lösungsmittel mit einem wasserlöslichen oder in Wasser ohne Hilfsmittel emulgierbaren Präpolymer vermischt wird, wobei das Präpolymer folgende Komponenten aufweist:
• eine zumindest über weite Sequenzen unpolare Hauptkette aus der Gruppe der fettsäuremodifizierten Polyester (Ölalkydharze),
• polare, hydrophile Gruppen, welche direkt oder in Seitenketten an der Hauptkette anhaften, aus der Gruppe der Carboxylat-, und Alkoxylgruppen,
• vernetzungsfähige Gruppen aus ein- bis mehrfach ungesättigten Fettsäureestern,
d) dass die Mischung gemäß Punkt a) mit Luftsauerstoff in Kontakt gebracht wird, sodass das Präpolymer über die vernetzungsfähigen Gruppen mittels Luftsauerstoff vernetzt, und
e) dass nach Abdunsten des wässrigen Lösungsmittels eine dreidimensional vernetzte, hydrophobe Polymermatrix gebildet wird, in welche die biologische Komponente eingebettet ist.

5. Verfahren nach Anspruch 4, **dadurch gekennzeichnet, dass** durch Erhöhung des Sauerstoffpartialdruckes auf Werte über 250 mb, vorzugsweise auf Werte über 500 mb, die Vernetzungstemperatur unter Raumtemperatur, vorzugsweise auf Temperaturen von 0 bis 5°C abgesenkt wird.

6. Verfahren nach einem der Ansprüche 1 bis 3, **dadurch gekennzeichnet, dass** als Präpolymer enaminvernetzende Präpolymere, vorzugsweise Polyacrylate oder Polymethacrylate verwendet werden.

7. Verfahren nach einem der Ansprüche 1 bis 6, **dadurch gekennzeichnet, dass** der Moleküldurchmesser der biologischen Komponente um mindestens einen Faktor 3, vorzugsweise um einen Faktor > 10 größer ist als die Maschenweite der dreidimensional vernetzten Polymermatrix, so dass die biologische Komponente migrationshemmend in der Polymermatrix zurückgehalten wird.

8. Verfahren nach einem der Ansprüche 1 bis 7, **dadurch gekennzeichnet, dass** als biologische Komponente ein Enzym aus der Gruppe der Oxidoreduktasen eingesetzt wird.

9. Verfahren nach Anspruch 9, **dadurch gekennzeichnet, dass** als Enzym Glucoseoxidase (EC 1.1.3.4), Lactatoxidase (EC 1.1.3.2) oder Ascorbatoxidase (EC 1.10.3.3) eingesetzt wird.

10. Verfahren nach einem der Ansprüche 1 bis 7, **dadurch gekennzeichnet, dass** als biologische Komponente ein Enzym aus der Gruppe der Hydrolasen eingesetzt wird.

11. Verfahren nach Anspruch 10, **dadurch gekennzeichnet, dass** als Enzym Urease (EC 3.5.1.5), Kreatinase (EC 3.5.3.3) oder Kreatininase (EC 3.5.4.21) eingesetzt wird.

12. Immobilisat, bestehend aus in einer Polymermatrix immobilisierten biologischen Komponenten, vorzugsweise Enzymen,
**dadurch gekennzeichnet, dass** das der Polymermatrix zugrundeliegende Präpolymer wasserlöslich oder in Wasser ohne Hilfsmittel emulgierbar ist und eine zumindest über weite Sequenzen unpolare Hauptkette aufweist, an welcher direkt oder in Seitenketten polare hydrophile Gruppen anhaften, sowie dass das Präpolymer über vernetzungsfähige Gruppen verfügt, welche ohne Anwendung von Katalysatoren oder energiereicher Strahlung bereits bei Temperaturen von Raumtemperatur bis 70°C, vorzugsweise bis 40°C, oder bei Zutritt von Luftsauerstoff reagieren und ein dreidimensionales Netzwerk bilden, in welches die biologische Komponente eingebettet ist.

13. Immobilisat nach Anspruch 12, **dadurch gekennzeichnet, dass** das Präpolymer ein Polymer aus der Gruppe der Polykondensations-, Polyadditions- und Polymerisationsharze ist, dessen Hauptkette zur Gruppe der Polyester, Polyamide, Epoxiharze, Phenolharze, Polyacrylate und Polymethacrylate gehört, deren polare, hydrophile Gruppen der Gruppe der Carboxylat-, Amino-, Ammonium-, Hydroxyl- und Alkoxylgruppen angehören und deren vernetzungsfähige Gruppe aus ein- bis mehrfach ungesättigten Fettsäureestern, β-Diketo-Gruppen sekundären Aminogruppen, geschützten Isocyanatgruppen, Epoxygruppen, Silanol- und Estergruppen bestehen.

14. Immobilisat nach Anspruch 12 oder 13, **dadurch gekennzeichnet, dass** die Hauptkette polare Sequenzen der Gruppe der Polyoxyalkylene, vorzugsweise Polyoxyethylen, der polymerisierten Acryl- und Methacrylsäuren sowie der Hydroxyalkylacrylate bzw. - methacrylate aufweist.

15. Immobilisat nach einem der Ansprüche 12 bis 14, **dadurch gekennzeichnet, dass** die Polymermatrix ein Ölalkydharz ein Polyacrylat oder ein Polymethacrylat ist.

16. Biosensor mit einem Immobilisat nach einem der Ansprüche 12 bis 15, **dadurch gekennzeichnet, dass** der Sensor eine Trägerschicht (4) aufweist, welche dahingehend chemisch modifiziert ist, dass diese zumindest eine dem Präpolymer des Immobilisates (S₁, S₂, S₃, S₄) analog vernetzungsfähige Gruppe zum Zwecke der Haftverbesserung zwischen Immobilisat und Trägerschicht (4) aufweist.

17. Biosensor nach Anspruch 16, **dadurch gekennzeichnet, dass** mehrere schichtartig angeordnete Immobilisate (S₁, S₂, S₃, S₄) vorgesehen sind, welche das gleiche Matrixmaterial (5) jedoch unterschiedliche biologische Komponenten (Enz₁, Enz₂, Enz₃) aufweisen.

18. Biosensor nach Anspruch 17, **dadurch gekennzeichnet, dass** das bzw. jedes der schichtartig angeordneten Immobilisate (S₁, S₂, S₃, S₄) mehrere biologische Komponenten beinhaltet.

19. Biosensor nach Anspruch 18, **dadurch gekennzeichnet, dass** mehrere biologische Komponenten in Form eines Multienzymsystems vorgesehen sind.

20. Biosensor nach einem der Ansprüche 16 bis 19, **dadurch gekennzeichnet, dass** in das Immobilisat (S₄) katalytische und/oder ladungsableitende Partikel eingelagert sind.

21. Biosensor nach Anspruch 20, **dadurch gekennzeichnet, dass** die katalytisch aktiven Partikel Edelmetallkolloide bzw. Edelmetallpigmente sind.

22. Biosensor nach Anspruch 20, **dadurch gekennzeichnet, dass** die katalytisch aktiven Partikel aus Manganoxid bestehen.

23. Biosensor nach einem der Ansprüche 20 bis 22, **dadurch gekennzeichnet, dass** die leitfähigen Partikel aus Graphit, Glaskohle oder Aktivkohle bestehen.

## Claims

1. Method for immobilizing biological components, preferably enzymes, in a polymer matrix, **characterized in that**
(a) at least one biological component in an aqueous solvent is mixed with a prepolymer which is water-soluble or can be emulsified in water without the use of an additive, said prepolymer comprising the following components:
• a main chain in which the large majority of sequences are nonpolar, and which belongs to the group of polyesters, polyamides, epoxy resins, phenolic resins, polyacrylates, and polymethacrylates,
• polar hydrophilic groups which are attached to the main chain either directly or in side chains, from the group of carboxylate, amino, ammonium, hydroxyl, and alkoxyl groups,
• cross-linking groups of β-diketo groups, secondary amino groups, protected isocyanate groups, epoxy groups, silanol and ester groups,
(b) and **in that** the mixture according to (a) is subject to temperatures ranging from room temperature to 70°C, and preferably not more than 40°C, so that the prepolymer will cure via the cross-linking groups without the use of catalysts or high-energy radiation,
(c) and **in that** a three-dimensional cross-linked hydrophobic polymer matrix is formed on evaporation of the aqueous solvent, in which the biological component is embedded.

2. Method according to claim 1, **characterized in that** peripheral regions of the biological component that have no influence on the function of the biological component take part in the cross-linking reaction of the prepolymer.

3. Method according to claim 1 or 2, **characterized in that** any polar sequences of the main chain belong to the group of polyoxyalkylenes, preferably polyoxyethylene, of the polymerized acrylic and methyl acrylic acids, and of the hydroxy alkylacrylates and -methacrylates.

4. Method for immobilizing biological components, preferably enzymes, in a polymer matrix, **characterized in that**
(a) at least one biological component in an aqueous solvent is mixed with a prepolymer which is water-soluble or can be emulsified in water without the use of an additive, said prepolymer comprising the following components:
• a main chain in which the large majority of sequences are nonpolar, and which belongs to the group of fatty acid modified polyesters (oil alkyd resins),
• polar hydrophilic groups which are attached to the main chain either directly or in side chains, from the group of carboxylate-, and alkoxyl groups,
• cross-linking groups of mono-unsaturated to poly-unsaturated fatty acid esters
(b) and **in that** the mixture according to (a) is brought into contact with atmospheric oxygen, so that the prepolymer will cure via the cross-linking groups by reaction with atmospheric oxygen,
(c) and **in that** a three-dimensional cross-linked hydrophobic polymer matrix is formed on evaporation of the aqueous solvent, in which the biological component is embedded.

5. Method according to claim 4, **characterized in that** by raising the oxygen partial pressure to more than 250 millibar, preferably to more than 500 millibar, the cross-linking temperature will be reduced to less than room temperature, i.e., preferably to 0-5°C.

6. Method according to any of claims 1 to 3, **characterized in that** enamine cross-linking prepolymers are used as prepolymers, preferably polyacrylates or polymethacrylates.

7. Method according to any of claims 1 to 6, **characterized in that** the molecule diameter of the biological component exceeds the mesh width of the three-dimensional cross-linked polymer matrix at least by a factor of 3, and preferably by a factor >10, so that the biological component is retained in the polymer matrix and thus prevented from migrating.

8. Method according to any of claims 1 to 7, **characterized in that** an enzyme from the group of oxidoreductases is used as biological component.

9. Method according to claim 8, **characterized in that** glucose oxidase (EC 1.1.3.4), lactic oxidase (EC 1.1.3.2), or ascorbate oxidase (EC 1.10.3.3) is used as enzyme.

10. Method according to any of claims 1 to 7, **characterized in that** an enzyme from the group of hydrolases is used as biological component.

11. Method according to claim 10, **characterized in that** urease (EC 3.5.1.5), creatinase (EC 3.5.3.3), or creatininase (EC 3.5.4.21) is used as enzyme.

12. Immobilization product, consisting of biological components, preferably enzymes, that are immobilized in a polymer matrix, **characterized in that** the basic prepolymer building the matrix is water-soluble or can be emulsified in water without the use of an additive, and has a main chain in which the large majority of sequences are nonpolar and to which polar hydrophilic groups are attached either directly or in side chains, and further **characterized in that** the prepolymer has cross-linking groups reacting without the use of catalysts or high-energy radiation at temperatures ranging from room temperature to 70°C, and preferably not exceeding 40°C, or upon contact with atmospheric oxygen, and forming a three-dimensional network in which the biological component is embedded.

13. Immobilization product according to claim 12, **characterized in that** the prepolymer is a polymer from the group of polycondensation, polyaddition, and polymerization resins, whose main chain belongs to the group of polyesters, polyamides, epoxy resins, phenolic resins, polyacrylates, and polymethacrylates, whose polar hydrophilic groups belong to the group of carboxylate, amino, ammonium, hydroxyl, and alkoxyl groups, and whose cross-linking group is composed of mono-unsaturated to poly-unsaturated fatty acid esters, β-diketo-groups, secondary amino groups, protected isocyanate groups, epoxy groups, silanol and ester groups.

14. Immobilization product according to claim 12 or 13, **characterized in that** any polar sequences of the main chain belong to the group of polyoxyalkylenes, preferably polyoxyethylene, of the polymerized acrylic and methyl acrylic acids, and of the hydroxy alkyl-acrylates and methacrylates.

15. Immobilization product according to any of claims 12 to 14, **characterized in that** the polymer matrix is an oil alkyd resin, a polyacrylate, or a polymethacrylate.

16. Biosensor made with the use of an immobilization product according to any of claims 12 to 15, **characterized in that** said sensor is provided with a supporting layer (4) which is chemically modified such that it exhibits at least one group whose cross-linking capacity matches that of the prepolymer of the immobilization product (S₁, S₂, S₃, S₄), in order to improve adhesion between immobilization product and supporting layer (4).

17. Biosensor according to claim 16, **characterized in that** a layered structure of several immobilization products (S₁, S₂, S₃, S₄) is provided, which have the same matrix material (5) but different biological components (Enz₁, Enz₂, Enz₃).

18. Biosensor according to claim 17, **characterized in that** the or each layered immobilization product (S₁, S₂, S₃, S₄) contains several biological components.

19. Biosensor according to claim 18, **characterized in that** several biological components are provided in the form of a multi-enzyme system.

20. Biosensor according to any of claims 16 to 19, **characterized in that** the immobilization product (S₄) contains catalytic and/or charge-conducting particles.

21. Biosensor according to claim 20, **characterized in that** the catalytically active particles are precious metal colloids or precious metal pigments.

22. Biosensor according to claim 20, **characterized in that** the catalytically active particles are of manganese oxide.

23. Biosensor according to any of claims 20 to 22, **characterized in that** the conducting particles are of graphite, glassy carbon, or activated charcoal.

## Revendications

1. Procédé pour immobiliser des composants biologiques, de préférence des enzymes, dans une matrice polymère, **caractérisé en ce que** :
a) au moins un composant biologique en solution aqueuse est mélangé avec un prépolymère hydrosoluble ou susceptible d'être émulsionné dans l'eau sans un adjuvant, le prépolymère présentant les composants suivants :
• une chaîne principale qui n'est pas polaire au moins sur des séquences importantes, choisie dans le groupe constitué par les polyesters, les polyamides, les résines époxy, les résines phénoliques, les polyacrylates et les polyméthacrylates,
• des groupes hydrophiles polaires portés directement par la chaîne principale ou par des chaînes latérales et choisis parmi les groupes carboxylate, amino, ammonium, hydroxyle et alcoxyle,
• des groupes capables de provoquer une réticulation, choisis parmi les groupes β-dicéto, amino secondaire, isocyanate protégé, époxy, silanol et ester,
b) le mélange selon a) est amené à une température située dans la plage allant de la température ambiante à 70 °C et, de préférence, à 40 °C, de sorte que le prépolymère est réticulé par la réaction des groupes capables de provoquer une réticulation, sans avoir à faire appel à des catalyseurs ou à des radiations à haut contenu énergétique et
c) après l'évaporation du solvant aqueux, une matrice polymère réticulée hydrophobe tridimensionnelle se trouve formée, dans laquelle le composant biologique se trouve emprisonné.

2. Procédé selon la revendication 1, **caractérisé en ce que**, dans la réaction de réticulation du prépolymère, sont également concernées des régions périphériques du composant biologique ne perturbant pas la fonction du composant biologique.

3. Procédé selon la revendication 1 ou la revendication 2, **caractérisé en ce que** la chaîne principale présente des séquences polaires choisies parmi les polyoxyalkylènes (de préférence des polyoxyéthylènes), l'acide acrylique polymérisé, l'acide méthacrylique polymérisé, ainsi que les hydroxyalkylacrylates polymérisés ou les hydroxyalkylméthacrylates polymérisés.

4. Procédé pour immobiliser des composants biologiques, de préférence des enzymes, dans une matrice polymère, **caractérisé en ce que** :
a) au moins un composant biologique en solution aqueuse est mélangé avec un prépolymère hydrosoluble ou susceptible d'être émulsionné dans l'eau sans un adjuvant, le prépolymère présentant les composants suivants :
• une chaîne principale qui n'est pas polaire au moins sur des séquences importantes, choisie parmi les polyesters modifiés par des acides gras (résines alkyde modifiées par une huile),
• des groupes hydrophiles polaires qui sont portés directement par la chaîne principale ou par des chaînes latérales, choisis parmi les groupes carboxylate et alcoxyle,
• des groupes capables de provoquer une réticulation, choisis parmi les esters d'acides gras présentant deux insaturations ou davantage,
d) le mélange selon a) est amené en contact avec l'oxygène de l'air, de sorte que le prépolymère est réticulé par la réaction des groupes capables de provoquer une réticulation, sous l'effet de l'oxygène de l'air, et
e) après l'évaporation du solvant aqueux, une matrice polymère réticulée hydrophobe tridimensionnelle est formée, dans laquelle le composant biologique se trouve emprisonné.

5. Procédé selon la revendication 4, **caractérisé en ce qu'**en augmentant la pression partielle de l'oxygène à une valeur dépassant 250 mb et, de préférence dépassant 500 mb, la température de réticulation est diminuée en dessous de la température ambiante et, de préférence, à une température de 0 à 5 °C.

6. Procédé selon l'une des revendications 1 à 3, **caractérisé en ce que** l'on utilise en tant que prépolymère, un prépolymère qui peut être réticulé par une énamine, et qui est, de préférence, un polyacrylate ou un polyméthacrylate.

7. Procédé selon l'une des revendications 1 à 6, **caractérisé en ce que** le diamètre des molécules du composant biologique est plus grand, par au moins un facteur de 3 et, de préférence un facteur > 10, que la maille de la matrice polymère réticulée tridimensionnelle, de sorte que la matrice polymère conserve sa capacité à empêcher une migration du composant biologique.

8. Procédé selon l'une des revendications 1 à 7, **caractérisé en ce que** l'on utilise en tant que composant biologique, une enzyme du groupe des oxydoréductases.

9. Procédé selon la revendication 8, **caractérisé en ce que** l'on utilise en tant qu'enzyme, la glucose oxydase (EC 1.1.3.4), la lactose oxydase (EC 1.1.3.2) ou l'ascorbate oxydase (EC 1.10.3.3).

10. Procédé selon l'une des revendications 1 à 7, **caractérisé en ce que** l'on utilise en tant que composant biologique, une enzyme du groupe des hydrolases.

11. Procédé selon la revendication 10, **caractérisé en ce que** l'on utilise en tant qu'enzyme, l'uréase (EC 3.5.1.5), la créatinase (EC 3.5.3.3) ou la créatininase (EC 3.5.4.21).

12. Immobilisat constitué par des composants biologiques immobilisés dans une matrice polymère, ces composants étant de préférence des enzymes, **caractérisé en ce que** le prépolymère dont est dérivée la matrice polymère est hydrosoluble ou susceptible d'être émulsionné dans l'eau sans un adjuvant, que le prépolymère a une chaîne principale qui n'est pas polaire au moins sur des séquences importantes, et qui porte des groupes hydrophiles polaires liés directement à cette chaîne principale ou liés à des chaînes latérales et que le prépolymère porte des groupes capables de provoquer une réticulation, qui réagissent, sans nécessiter un catalyseur ou des radiations à haut contenu énergétique, déjà à une température située dans la plage allant de la température ambiante à 70 °C et, de préférence à 40 °C, ou en présence de l'oxygène de l'air, pour constituer un réseau tridimensionnel, dans lequel le composant biologique se trouve emprisonné.

13. Immobilisat selon la revendication 12, **caractérisé en ce que** le prépolymère est un polymère choisi parmi les résines de polycondensation, de polyaddition et de polymérisation, dont la chaîne principale est choisie parmi les polyesters, les polyamides, les résines époxy, les résines phénoliques, les polyacrylates et les polyméthacrylates, dont les groupes hydrophiles polaires sont choisis parmi les groupes carboxylate, amino, ammonium, hydroxyle et alcoxyle et, dont les groupes capables de provoquer une réticulation sont des esters d'acides gras portant une insaturation ou davantage, les groupes β-dicéto, amino secondaire, isocyanate protégé, époxy, silanol et ester.

14. Immobilisat selon la revendication 12 ou la revendication 13, **caractérisé en ce que** les séquences polaires de la chaîne principale sont choisies dans le groupe des polyoxyalkylènes (de préférence, les polyoxyéthylènes), de l'acide acrylique polymérisé, de l'acide méthacrylique polymérisé, ainsi que des hydroxyalkylacrylates polymérisés et des hydroxyalkylméthacrylates polymérisés.

15. Immobilisat selon l'une des revendications 12 à 14, **caractérisé en ce que** la matrice polymère est une résine alkyde modifiée par une huile, un polyacrylate ou un polyméthacrylate.

16. Biodétecteur avec un immobilisat selon l'une des revendications 12 à 15, **caractérisé en ce que** le détecteur a une couche de support (4) qui est modifiée chimiquement pour avoir un / des groupes capables de réticuler analogues à celui */* ceux du prépolymère des immobilisats (S₁, S₂, S₃, S₄), pour améliorer l'adhésion entre l'immobilisat et la couche de support (4).

17. Biodétecteur selon la revendication 16, **caractérisé en ce que** l'on prévoit plusieurs immobilisats (S₁, S₂, S₃, S₄) disposés en couches qui sont constituées par le même matériau matriciel (5), mais qui contiennent des composants biologiques différents (Enz₁, Enz₂, Enz₃).

18. Biodétecteur selon la revendication 17, **caractérisé en ce qu'**un ou plusieurs des immobilisats (S₁, S₂, S₃, S₄) disposés en couches contient ou contiennent plusieurs composants biologiques.

19. Biodétecteur selon la revendication 18, **caractérisé en ce que** l'on prévoit plusieurs composants biologiques, sous la forme d'un système multi-enzymatique.

20. Biodétecteur selon l'une des revendications 16 à 19, **caractérisé en ce que** l'immobilisat (S₄) comprend des particules catalytiques et / ou conductrices de charges.

21. Biodétecteur selon la revendication 20, **caractérisé en ce que** les particules ayant une activité catalytiques sont constituées par un métal rare colloïdal ou par un pigment de métal rare.

22. Biodétecteur selon la revendication 20, **caractérisé en ce que** les particules ayant une activité catalytique sont constituées d'oxyde de manganèse.

23. Biodétecteur selon l'une des revendications 20 à 22, **caractérisé en ce que** les particules conductrices sont constituées de graphite, de carbone vitreux ou de charbon actif.
